# EUROPEAN PATENT APPLICATION

(11) **EP 1 813 944 A1**
(43) Date of publication of application: **01.08.2007**
(21) Application number: 05788052.8
(22) Date of filing: 03.10.2005
(51) Int. Cl.: G01N 33/53, C12N 15/09, G01N 33/574, G01N 33/577

(54) **DRUG FOR MONITORING WORSENING OF HEPATITIS**

(30) Priority: 05.10.2004 JP 2004292791
(71) Applicant: Perseus Proteomics Inc., Tokyo 153-0041 (JP); The University of Tokyo, Bunkyo-Ku, Tokyo 113-8654 (JP)
(72) Inventor: NAKAJIMA, Atsushi, Tokyo,108-0074 (JP); ABURATANI, Hiroyuki, Tokyo,180-0003 (JP); IWANARI, Hiroko c/o Perseus Proteomics Inc.,, Tokyo; 153 0041 (JP); OHKUCHI, Masao c/o Perseus Proteomics Inc., Park, 1530 041 (JP); SATOH, Hirokazu c/o Perseus Proteomics Inc.,, Tokyo; 153 0041 (JP)
(74) Representative: Blodig, Wolfgang
(86) International application number: PCT/JP2005/018290
(87) International publication number: WO 2006/038588

(57) **Abstract**

The present invention relates to a method of monitoring seriousness of hepatitis, comprising measuring GPC3 using an antibody which recognizes an N-terminal region of GPC3. According to the present invention, seriousness of hepatitis, specifically, progression of hepatitis or cirrhosis toward hepatoma can be presumed.

## Description

### Technical field

The present invention relates to a method of monitoring seriousness of hepatitis, and a monitoring agent therefor.

### Background art

A primary hepatocellular carcinoma (hereinafter, referred to as hepatoma) is known to mainly attack patients infected with hepatitis B virus and hepatitis C virus after progression from chronic hepatitis to cirrhosis. Among the patients with the viral infection, the patients infected with the hepatitis C virus particularly have a high possibility of progression toward the hepatoma, and its onset tends to increase in recent years. As the therapeutic means of the hepatoma, hepatectomy, percutaneous local treatments (radiofrequency ablation, ethanol injection therapy and the like), transcatheter arterial embolization therapy (TAE), continuous arterial infusion chemotherapy, radiation therapy and the like have been used, but there are many cases where these therapies do not lead to a complete cure. Accordingly, for the purpose of medical treatments for preventing progression toward hepatoma by virus removal and immunopotentiation on chronic hepatitis and cirrhosis which is at a stage of progression toward hepatoma, in recent years, various antiviral agent and interferons have been developed and used in clinical applications.

At present, AST (GOT), ALT (GPT), and γ-GTP (γ-glutamyl transpeptidase) are generally used in examination for aberration in liver, but in addition to the above, TP (serum total protein), A/G (albumin/globulin ratio), UA (urea), T-Cho (total cholesterol), TTT (thymol turbidity test), ZTT (zinc sulfate turbidity test), LDH (lactate dehydrogenase), ALP (alkaline phosphatase) and chE (cholinesterase) are used as indexes for aberration in liver. Although aberration in liver is diagnosed by such many examinations, further a virus test is carried out, and the extent of progressionof inflammation, filamentation and malignant transformation is diagnosed by a biopsy.

The progression from virus infection, hepatitis, chronic hepatitis, and cirrhosis toward hepatoma is diagnosed at present stage by diagnosing the progression from inflammation and filamentation toward malignant transformation by an examination using various markers and an examination using a biopsy. Not only since such treatment is a burden to a patient and further a problem in view of medical economy, but also in order to obtain suitable medical index, it is desired to develop a method of simply presuming the onset of hepatoma.

Existence of glypican family as a new family of heparan sulfate proteoglycan which exists on a cell surface has been reported. Up to now, it has been reported that six kinds of glypican (glypican 1, glypican 2, glypican 3, glypican 4, glypican 5 and glypican 6) exist as members of glypican family. The members of this family have a core protein of a uniform size (about 60 kDa) and commonly have a specific and well-conserved sequence of cysteine, and are bound to a cytoplasmic membrane by glycosylphosphatidylinositol (GPI) anchor.

Glypican 3 (GPC3) is known to deeply relate to cell division in development and a pattern control thereof. GPC3 is known to be secreted in a blood, and Non-patent document 1 suggested that it is cleaved between amino acid 358 and amino acid 359. On SDS-PAGE performed under a reduction condition, GPC3 is divided into a fragment from amino acid 1 to 358 (N-terminal) and a fragment from amino acid 359 to 580 (C-terminal) which has become a macromolecule by addition of heparan sulfate chain.

On the other hand, it is known that GPC3 gene highly expresses in a hepatoma cell and may be utilized as amaker for hepatocellular carcinoma. Further, the methods of diagnosing hepatoma by measuring GPC3 are already known in Patent documents 1 to 3 and Non-patent document 2.

For conventional agents for measuring GPC3, Patent document 1 using an antibody for recognizing a C-terminal region of GPC3 disclosed that a positive rates thereof in hepatoma, in a healthy individual and chronic hepatitis, and in cirrhosis were 53%, the detection limitation or less, and 5%, respectively. Patent document 2 using an antibody for recognizing the region in the vicinity of N-terminal/ C-terminal cleavage disclosed that a positive rate thereof was 40% in hepatoma, and 0% in a healthy individual and chronic hepatitis. Additionally, Non-patent document 2 using an antibody for recognizing N-terminal disclosed that a positive rate thereof was 73% after hepatoma TAE and 53% after surgical operation for hepatoma, and indicated that the antibody is useful as an agent for diagnosing hepatoma because its positive rate was significant different from cirrhosis. However, it has not been known how GPC3 changes before onset of hepatoma.
[Patent document 1] WO 03/10042
[Patent document 2] WO 2004/018667
[Patent document 3] WO 2004/038420
[Non-patent document 1] J. Cell. Biol. , 163 (3) : 625-35. 2003
[Non-patent document 2] CANCER RESEARCH 64, 1-6, April 1, 2004

### Disclosure of invention

### Problem to be solved by the Invention

An object of the present invention is to provide means for monitoring seriousness of hepatitis. Means for solving the problem

The present inventors developed a novel antibody which was an antibody not for recognizing a C-terminal region or the region in the vicinity of N-terminal/ C-terminal cleavage but for recognizing an N-terminal region, and measured the GPC3 concentration in the serum of patients with hepatitis and cirrhosis which is at a stage before onset of hepatoma. As the result of measuring the GPC3 concentration in the serum of patients with liver disease according to its measurement system, its positive rate with a cutoff value of 0.4 was 1.5% in a healthy individual, while it was higher in chronic hepatitis (36%) and in cirrhosis (51.4%). On the other hand, the positive ratio was 51% in a hepatoma patient. Further, immunostaining of tissue from the liver of a chronic hepatitis patient and a cirrhosis patient showed positive in growth hepatocyte. This led to the finding that new GPC3 measuring system of the present invention is useful as an agent for presuming progression from hepatitis or cirrhosis to hepatoma but not for diagnosing hepatoma, that is, an agent for monitoring seriousness of hepatitis, thereby completing the present invention.

That is, the present invention provides an agent for monitoring seriousness of hepatitis, wherein the agent comprises an anti-GPC3 antibody.
Also, the present invention provides a method of monitoring seriousness of hepatitis, comprising measuring GPC3 in a sample using an anti-GPC3 antibody.

### Effect of invention

The present invention can provide a simple method for monitoring seriousness of hepatitis which has been difficult to be predicted by any single conventional agents for detecting aberration in liver, whereby new therapeutic regimen can be made rapidly.

### Brief Description of the Drawings

Fig. 1 shows western blotting for confirming the recognition site of GPC3 antibody.
Fig. 2 is a graph showing standard curves obtained by antibodies for recognizing C-terminal and N-terminal.

### Best Mode for Carrying out the Invention

The present invention will be described below in details.
The present invention relates to a method of monitoring seriousness of hepatitis by measuring GPC3 in a sample using an anti-GPC3 antibody.

The term "measurement" includes a quantitative or non-quantitative measurement. For example, the non-quantitative measurement includes a simple measurement as to whether or not GPC3 exists, a measurement as to whether or not GPC3 exists in a certain amount or more, a measurement for comparing the amount of GPC3 in the sample with other samples (for example, a control sample or the like), and the like; and the quantitative measurement includes a measurement of the concentration of GPC3, a measurement of the amount of GPC3, and the like.

The term "sample" is not particularly limited so long as it can contain GPC3, but a sample obtained from the living body such as a mammal is preferable, and a sample obtained from human is more preferable. As the specific examples of the sample include blood, interstitial fluid, plasma, extravascular fluid, cerebrospinal fluid, synovial fluid, pleural fluid, serum, lymph, saliva, urine, and the like, and blood, serum and plasma are preferable. More preferable is blood, serum and plasma obtained from a patient with hepatitis, particularly with chronic hepatitis or cirrhosis. In addition, the sample of the present invention also includes a sample derived from a cell culture obtained from the living body, or the like.

As the anti-GPC3 antibody used in the present invention, any of monoclonal antibodies and polyclonal antibodies may be used. In addition, an antibody against secretory GPC3, that is anti-soluble GPC3 antibody, is preferable as the anti-GPC3 antibody, since blood, serum or plasma is used as the sample. As the polyclonal antibody, antiserum is preferably used. It is more preferable to use anti-soluble GPC3 monoclonal antibody for recognizing an N-terminal region. A case of using anti-GPC3 monoclonal antibody will be described below in detail.

### 1. Preparation of anti-GPC3 monoclonal antibody

So long as the anti-GPC3 monoclonal antibody used in the present invention specificallybinds to GPC3 protein, its origin and form are not limited. Specifically, any know monoclonal antibodies can be used, such as avian antibodies, murine antibodies, rat antibodies, human antibodies, chimera antibodies, humanized antibodies, or the like.
In addition, it is preferable that the anti-GPC3 monoclonal antibody immobilized on a support and the anti-GPC3 monoclonal antibody labeled by a labeling substance recognize different epitopes of GPC3 molecules, respectively. The recognition site is not particularly limited, but an antibody for recognizing the N-terminal is preferable.

The anti-GPC3 monoclonal antibody used in the present invention can be produced by using any known means. As the anti-GPC3 monoclonal antibody used in the present invention, monoclonal antibodies derived from mammal and avian are preferable. The monoclonal antibodies derived from mammal and avian include those produced by hybridoma, and those produced in a host which is transformed with an expression vector containing genes of the antibody by a genetic engineering technique.

A hybridoma which produces the monoclonal antibody can be prepared as described below, basically using known techniques. Specifically, GPC3 or soluble GPC3 and a segment peptide thereof are used as an immunogen according to a usual immunization method, the obtained immunocyte is then fused with a known parental cell by a conventional cell fusion method, and a monoclonal antibody producing cell is screened according to a conventional screening method, whereby the monoclonal antibody producing hybridoma can be produced.

Specifically, the monoclonal antibody may be prepared as follows.
At first, the GPC3 used as the immunogen for producing an antibody can be obtained by culturing HuH6 or HepG2 cell strain or the like and purifying natural GPC3 contained in a supernatant thereof. Also, it can be obtained by expressing GPC3 (MXR7) gene/amino acid sequence as disclosed by Lage, H. et al., Gene 188 (1997), 151-156. That is, after inserting the gene sequence encoding GPC3 into a known expression vector system to transform a suitable host cell, a target recombinant GPC3 protein is purified from the host cell or a culture supernatant thereof by any known methods.
Next, the purified GPC3 is used as an immunogen. The partial peptide of GPC3 can be used as the immunogen. In this case, the partial peptide can be obtained from the amino acid sequence of human GPC3 by chemical synthesis, it can be also obtained by incorporating a part of GPC gene into an expression vector, and it can be further obtained by degradating natural GPC3 by means of proteolytic enzyme. The region and size of GPC3 to be used as the partial peptide is not limited. In the present invention, it is preferable that full-length GPC3 is used as the immunogen.

The mammals and avians immunized with an immunogen are not particularly limited, and preferably selected in view of adaptability to parental cells used in cell fusion. Generally, rodents (e. g., mouse, rat, or hamster), guinea pig, rabbit, monkey, and chicken are used.

Immunization of animals with the immunogen is performed according to any known methods. For example, as a general method, it is performed by interperitoneal injection or subcutaneous injection of the immunogen to the animals. Specifically, the sensitized antigen is diluted and suspended with a suitable amount of PBS (Phosphate-Buffered Saline), physiological saline or the like, then if desired, the resultant solution is mixed with a suitable amount of a conventional adjuvant, for example, Freund's complete adjuvant, and after emulsification, it is administered to animals several times every 4 to 21 days. In addition, at the time of immunization with the immunogen, a suitable carrier can be also used. Particularly, in the case of using a partial peptide having a small molecular weight as the immunogen, it is preferable to bind it to a carrier protein such as albumin, keyhole limpet hemocyanin, or the like for immunization.

The animals are immunized in this manner, the increase in the level of a desired antibody in the serum is confirmed, and then an immunocyte is collected from the animals to use in cell fusion. A particular example of preferable immunocyte includes splenocyte.

As a parent cell for fusing with the above-described immunocyte, myeloma cells from animals are used. The myeloma cells suitably used is various known cell strains, for example, P3 (P3 × 63Ag8.653) (J. Immnol. (1979) 123, 1548-1550), P3x63Ag8U.1 (Current Topics in Microbiology and Immunology (1978) 81, 1-7), NS-1 (Kohler. G. and Milstein, C. Eur. J. Immunol. (1976) 6, 511-519), MPC-11 (Margulies. D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I. S. J. Exp. Med. (1978) 148, 313-323), R210 (Galfre, G. et al., Nature (1979) 277, 131-133).

The cell fusion of the above-described immunocyte and the myeloma cell can be performed basically according to any known methods, for example, methods of Kohler and Milstein (Kohler. G. and Milstein, C., Methods Enzymol. (1981) 73, 3-46).

More specifically, the above-described cell fusion is performed in a usual nutrient culture solution, for example, under the presence of a cell fusion accelerator. As the cell fusion accelerator, for example, polyethylene glycol (PEG), or Hemagglutinating Virus of Japan (HVJ) are used, and further if desired, an adjuvant such as dimethyl sulfoxide can be added to increase fusion efficiency.

The use ratio of the immunocyte and the myeloma cell can be arbitrarily set. For example, it is preferable that the immunocyte is used in an amount 1 to 10 times larger than that of the myeloma cell. The culture solution which can be used for the above-described cell fusion includes, for example, RPMI1640 culture solution which is suitable for growth of the above-described myeloma cell strain, MEM culture solution, and other usual culture solutions used in such cell culture, and further it can be combined with a serum supplement such as fetal calf serum (FCS).

The cell fusion is performed by fully mixing the predetermined amounts of the immunocyte and the myeloma cell in the above-described culture solution, adding PEG solution (for example, average molecular weight of about 1,000 to 6, 000) previously heated to about 37°C, at a concentration of usually 30 to 60 % (w/v), and mixing the solutions to form a target fused cell (hybridoma). Next, the mixture is repeatedly added a suitable culture solution sequentially and centrifuged to remove a supernatant, thereby cell fusing reagents which are not preferable for growth of the hybridoma are removed.

The obtained hybridoma is selected by culturing in a usual selective culture solution, for example, HAT culture solution (culture solution containing hypoxanthine, aminopterin and thymidine). The culture in the above-described HAT culture solution is continued for a sufficient time (usually, several days to several weeks) to kill cells other than target hybridoma (non-fused cells). Next, a usual limiting dilution method is performed, and hybridoma producing a target antibody is subjected to screening and single cloning.

The screening and single cloning of the target antibody may be performed by a screening method based on known antigen-antibody reaction. For example, an antigen is bound to a support such as beads formed of polystyrene or the like, or a commercially available 96-well microtiter plates, then reacted with the supernatant of hybridoma culture, and after washing the support, it is reacted with an enzyme labeled secondary antibody or the like, whereby it is possible to determine whether the target antibody capable of reacting with the immunogen is contained in a culture supernatant. The hybridoma producing the target antibody can be cloned by a limiting dilution method or the like. In this case, the same antigen as used for the immunization can be used as the antigen.

In addition to obtaining the above-described hybridoma by immunizing animals other than human with an antigen, a desired human antibody having avidity to GPC3 can be obtained by sensitizing a human lymphocyte in vitro to GPC3, and fusing the sensitized lymphocyte with a human-derived myeloma cell having permanent division potential (see Japanese Patent Publication No. H01-59878). Further, GPC3 which functions as an antigen is administered to a transgenic animal having all repertory of human antibody genes, then anti-GPC3 monoclonal antibody producing cell is collected and immortalized, and a human antibody against GPC3 may be obtained from the immortalized cell (see WO 94/25585, WO 93/12227, WO 92/03918 and WO 94/02602).

The prepared hybridoma producing a monoclonal antibody can be subcultured in a usual culture medium, and it can be preserved in liquid nitrogen for a long period of time.

In order to obtain a monoclonal antibody from the hybridoma, a method comprising culturing the hybridoma according to a usual manner and obtaining the monoclonal antibody as the culture supernatant, a method comprising administering the hybridoma to a mammal compatible thereto, growing the hybridoma therein, and obtaining the monoclonal antibody from ascites, or the like can be employed. The former method is suitable for obtaining the antibody in high purity, and the latter method is suitable for mass production of the antibody.

As a monoclonal antibody of the present invention, a recombinant monoclonal antibody can be used which is obtained by cloning an antibody gene from hybridoma, incorporating the gene into a proper vector, introducing this vector into a host, and producing the monoclonal antibody using a gene recombination technique (for example, see Vandamme, A. M. et al., Eur. J. Biochem. (1990) 192, 767-775, 1990). Specifically, mRNA which encodes the variation (V) regionof anti-GPC3 monoclonal antibody is isolated from hybridoma producing anti-GPC3 monoclonal antibody. The isolation of mRNA is performed by any known methods, for example, guanidine ultracentrifugation method (Chirgwin, J. M. et al., Biochemistry (1979) 18, 5294-5299), AGPC method (Chomczynski, P. et al., Anal. Biochem. (1987) 162, 156-159), and the like to prepare total RNA, from which target mRNA is prepared using mRNA Purification Kit (manufactured by Pharmacia). Alternatively, mRNA can be directly prepared with use of QuickPrep mRNA Purification Kit (manufactured by Pharmacia).

cDNA of antibody variable (V) region is synthesized from the obtained mRNA with use of a reverse transcriptase. The synthesis of the cDNA is performed by using AMV Reverse Transcriptase First-strand cDNA Synthesis Kit (manufactured by Biochemical Industry Inc.) or the like. In order to perform the synthesis and amplification of cDNA, 5' -RACE method (Frohman, M. A. et al., Proc. Natl. Acad. Sci. USA (1988) 85, 8998-9002, Belyavsky, A. et al. , Nucleic Acids Res. (1989) 17, 2919-2932) using 5'-Ampli FINDER RACE Kit (manufactured by Clontech) and PCR, or the like can be used.

Target DNA fragment is purified from the obtained PCR product and ligated to a vector DNA. Further, it is used to prepare a recombinant vector and introduced to Escherichia coli or the like, then colony is selected and a desired recombinant vector is prepared. Next, the base sequence of target DNA is identified by any known methods, for example, dideoxynucleotide chain termination method or the like.

After obtaining DNA which encodes the V-region of the aimed anti-GPC3 monoclonal antibody, the DNA is integrated into an expression vector containing DNA which encodes a desired antibody constant region (C-region).

In order to produce the anti-GPC3 monoclonal antibody used in the present invention, the antibody gene is integrated into an expression vector such that the antibody gene is expressed under control of expression control regions, for example, enhancer or promoter. Next, by this expression vector, a host cell is transformed and the antibody is expressed.

The expression of the antibody gene may be performed by separately incorporating DNA for encoding an antibody heavy chain (H-chain) and those encoding an antibody light chain (L-chain) into separate expression vectors and concurrently transforming host cells, or by incorporating DNA for encoding H-chain and those encoding L-chain into a single expression vector and transforming a host cell (see WO 94/11523).

In addition, not only the above-described host cell but also a transgenic animal can be used for production of a recombinant antibody. For example, an antibody gene is inserted into a gene encoding protein intrinsically produced in milk (goat β-casein, or the like), to prepare a fused gene. DNA fragment containing the fused gene into which the antibody gene is inserted is injected to an embryo of goat, and this embryo is implanted into a female goat. A desired antibody is obtained from milk produced by a transgenic goat born from the goat receiving the embryo or descendants thereof. In order to increase the amount of the milk containing the desired antibody produced by a transgenic goat, suitable hormone may be used for the transgenic goat ( Ebert, K. M. et al., Bio/Technology (1994) 12, 699-702).

In the present invention, in addition to the above-described antibody, an artificially modified genetic recombinant antibody, for example, a chimera antibody and a humanized antibody, can be used. These modified antibodies can be produced with use of any known methods.

The chimera antibody is obtained by ligating an antibody V-region encoding DNA as obtained above with a human antibody C-region encoding DNA, integrating this ligated DNA into an expression vector, and introducing the vector to a host. With use of this known method, the chimera antibody useful for the present invention can be obtained.

The humanized antibody is also referred to as reshaped human antibody, and this is obtained by grafting the complementarity determining region (CDR) of antibody of mammals other than human (for example, mouse) into the CDR of human antibody, and a general method for such genetic recombination is known (see EP 125023 and WO 96/02576).

Specifically, DNA sequence which is designed so as to ligate CDR of a murine antibody with framework region (FR) of human antibody is synthesized by PCR method using, as a primer, some oligonucleotides prepared to have overlapping sites in terminal regions of both CDR and FR (see the method disclosed in WO98/13388).

The FRs of a human antibody to be ligated through CDR is selected from one with which the CDRs form a good antigen binding site. According to necessity, the amino acid of the FRs in the V region of an antibody may be substituted such that the CDR of a reshaped human antibody forms a suitable antigen binding site (Sato, K. et al., Cancer Res. (1993) 53, 851-856).

In the C-region of a chimera antibody and a humanized antibody, the C-region of a human antibody is used. For example, Cγ1, Cγ2, Cγ3 and Cγ4 can be used for H-chain, while Cκ and Cλ can be used for L-chain. In addition, in order to improve the stabilityof the antibody or production thereof, the C-region of the human antibody may be modified.

The chimera antibody is composed of the V region of an antibody derived from mammals other than human and the C-region derived from human antibody. On the other hand, the humanized antibody is composed of the CDR of an antibody derived from mammals other than human, and the C-region and FR derived from human antibody.

The antibody used in the present invention is not limited to the whole molecule of an antibody, but may be the fragment of an antibody or a modified fragment thereof, and also includes a divalent antibody and a monovalent antibody as long as it binds to GPC3. For example, the antibody fragment includes Fab, F(ab')2, Fv, Fab/c having one Fab and complete Fc, or single chain Fv (scFv) ligating Fv of H-chain or L-chain with a suitable linker. Specifically, it is obtained by treating an antibody with an enzyme, for example, papain or pepsin to form an antibody fragment, or alternatively by constructing a gene encoding these antibody fragments, introducing it to an expression vector and then expressing it in a suitable host cell (For example, see Co, M. S. et al., J. Immunol. (1994) 152, 2968-2976, Better, M. & Horwitz, A. H. Methods in Enzymology (1989) 178, 476-496, Academic Press, Inc., Plueckthun, A. & Skerra, A. Methods in Enzymology (1989) 178, 476-496, Academic Press, Inc., Lamoyi, E., Methods in Enzymology (1989) 121, 652-663, Rousseaux, J. et al., Methods in Enzymology (1989) 121, 663-669,Bird, R. E. et al., TIBTECH (1991) 9, 132-137).

scFv is obtained by ligating the V-region of antibody H-chain and V-region of antibody L-chain. In this scFv, the V-region of the H-chain and the V-region of the L-chain are linked through a linker, preferably a peptide inker (Huston, J. S. et al., Proc. Natl. Acad. Sci. U.S.A. (1988) 85, 5879-5883). The V-region of the H-chain and the V-region of the L-chain in scFv may be derived from any antibodies described in the present specification. As the peptide linker linking the V-regions, for example, any single-strand peptides composed of 12 to 19 amino acid residues are used.

DNA encoding scFv is obtained by making DNA encoding all or desired amino acids sequences derived from DNA encoding the H-chain of the antibody or its V-region and those encoding the L-chain of the antibody or its V-region as a template, amplifying them by PCR using a pair of primers defining both ends thereof, and then amplifying DNA encoding a peptide linker using a pair of primers which defines both terminals thereof to be linked to the H-chain and the L-chain, respectively.

In addition, once DNAs for encoding scFv are prepared, an expression vector containing them, and a host transformed by the expression vector can be obtained by usual methods, and its host can be used to obtain scFv according to usual methods.

These antibody fragments can be expressed by obtaining their gene as in the above-described, and producing them in a host. The "antibody" in the present invention includes the fragments thereof.

As the modified form of an antibody, anti-GPC3 bound to various molecules such as labeled substances can be also used. The "antibody" in the present invention includes these modified forms of the antibody. Such modified form of the antibody can be obtained by subjecting the antibody obtained to chemical modification. The method of modifying an antibody has been already established in the art.

Further, the antibody used in the present invention may be also a bispecific antibody. The bispecific antibody may be those having an antigen binding site for recognizing different epitopes on GPC3 molecule, or those having one antigen binding site for recognizing GPC3 and the other antigen binding site for recognizing a labeled substance or the like. The bispecific antibody can be prepared by bonding a pair of HLs derived from two kinds of antibodies, or obtained by fusing hybridomas each producing different monoclonal antibody to produce a fusion cell producing bispecific antibody. Further, the bispecific antibody can be prepared according to a genetic engineering procedure.

The antibody gene constructed as described above can be expressed and obtained according to any known methods. In the case of a mammal cell, a commonly used and useful promoter, an antibody gene to be expressed, and poly A signal at 3' -side downstream thereof can be operably ligated to be expressed. For example, the promoter/enhancer can include human cytomegalovirus immediate early promoter/enhancer.

Another promoter/enhancer which can be used for antibody expression in the present invention includes virus promoter/enhancer such as retrovirus, polyoma virus, adenovirus, simian virus 40 (SV40), and mammal cell-derived promoter/enhancer such as human elongation factor 1a (HEF1a).

The gene expression can be easily performed using SV40 promoter/enhancer according to a method of Mulligan et al. (Nature (1979) 277, 108), and the gene expression can be easily performed using HEF1a promoter/enhancer according to a method of Mizushima et al. (Nucleic Acids Res. (1990) 18, 5322).

In the case of Escherichia coli, a commonly used and useful promoter, a signal sequence for antibody secretion and an antibody gene to be expressed can be operably ligated to express the gene. The promoter can include for example, lacz promoter and araB promoter. In the case of using the lacz promoter, the gene can be expressed according to a method of Ward et al. (Nature (1989) 341, 544-546; FASEB J. (1992) 6, 2422-2427), or in the case of using the araB promoter, the gene can be expressed according to a method of Better et al. Science (1988) 240, 1041-1043).

As the signal sequence for antibody secretion, if producing it in the periplasm of Escherichia coli, pelB signal sequence (Lei, S. P. et al J. Bacteriol. (1987) 169, 4379) may be used. Next, after separating the antibody produced in the periplasm, the structure of the antibody is suitably refolded and used.

The replication origin which can be used is one derived from SV40, polyoma virus, adenovirus, bovine papiroma virus (BPV) or the like. Further, an expression vector for amplification of the copy of the gene in a host cell system can contain aminoglycoside transferase (APH) gene, thymidine kinase (TK) gene, Escherichia coli xanthine guanine phosphoribosyl transferase (Ecogpt) gene, dihydrofolic acid reduction enzyme (dhfr) gene and the like, as a selective marker.

In order to produce the antibody used in the present invention, any expression systems, for example, eukaryotic cell system or prokaryotic cell system can be used. The eukaryotic cell includes, for example, animal cells such as established mammal cell system, insect cell system, mycotic cell and yeast cell, and the prokaryotic cell includes, for example, bacterial cell such as Escherichia coli cell.

Preferably, the antibody used in the invention is expressed in a mammalian cell, for example, CHO, COS, myeloma, BHK, Vero or HeLa cell.
Next, the transformed host cell is cultured in vitro or in vivo to produce a target antibody. The culture of the host cell is performed according to any known methods. For example, as a culture solution, DMEM, MEM, RPMI1640 and IMDM can be used, and a serum supplement such as fetal calf serum (FCS) can be used in combination.

The antibody expressed and produced as described above can be separated from a cell or a host animal to be purified to uniform state. The separation and purification of the antibody used in the present invention can be performed with affinity column. For example, the column using protein A column includes Hyper D, POROS, Sepharose F.F. (manufactured by Pharmacia) and the like. Another usual methods used for separation and purification of proteins may be used and these methods are not particularly limited. For example, chromatography columns other than the above-described affinity column, filter, ultrafiltration, salting out, dialysis, and the like can be suitably selected and combined to separate and purify the antibody (Antibodies A Laboratory Manual. Ed Harlow, David Lane, Cold Spring Harbor Laboratory, 1988).

The antibody epitope used in the present invention may be those recognizing the full-length GPC3 or a peptide fragment thereof, but preferably an antibody for recognizing an N-terminal region of GPC3 is desired.

### 2. Measurement of GPC3

GPC3 measured in the present invention may be GPC3 protein attaching heparan sulfate or the like, or GPC3 core protein.
Measurement of GPC3 contained in a sample is measured by an immunological method using anti-GPC3 antibody. The immunological method includes for example, radioimmunoassay, enzymeimmunoassay, fluoroimmunoassay, luminescence immunoassay, immunoprecipitation, immunonephelometry, western blotting, immunostaining and immunodiffusion, preferably enzymeimmunoassay, and particularly preferable is enzyme-linked immunosorbent assay (ELISA) (for example, sandwich ELISA). The above-described immunological method such as ELISA can be performed according to a method known by a skilled person in the art.

The general measurement method using anti-GPC3 antibody includes, for example, a method of immobilizing anti-GPC3 antibody to a support, adding a sample thereto, incubating it to bind anti-GPC3 antibody and GPC3 protein, washing them, and measuring the GPC3 protein bound to the support through the anti-GPC3 antibody to perform the measurement of the GPC3 protein in the sample.

The support used in the present invention includes, for example, insoluble support, such as insoluble polysaccharides such as agarose and cellulose; synthetic resins such as silicone resin, polystyrene resin, polyacrylamide resin, nylon resin and polycarbonate resin; and glass. These supports can be used in a form of beads, plate, or the like. In the case of beads, a column charged with the beads can be used. In the case of a plate, a multi-well plate (96-well multi-well plate and the like), a biosensor chip or the like can be used. The binding of anti-GPC3 antibody and the support can be performed according to any usual methods such as a chemical binding method or a physical adsorption method. As these support, commercially available support can be used.

The binding of anti-GPC3 antibody and GPC3 protein is usually performed in a buffer solution. As the buffer solution, a phosphoric acid buffer solution, a Tris buffer solution, a citric acid buffer solution, a borate buffer solution, a carbonate buffer solution or the like is used, for example. Incubation is performed under any conventional well used condition, for example, for 1 hour to 24 hours at 4°C to room temperature. Washing after incubation is not limited as long as it does not hinder the binding of GPC3 protein and anti-GPC3 antibody, and for example, a buffer solution containing a surfactant such as Tween 20 or the like is used.

In the GPC3 protein measuring method of the present invention, in addition to a sample to be measured GPC3 protein therein, a control sample may be used. The control sample includes a negative control sample not containing GPC3 protein, and a positive control sample containing GPC3 protein, or the like. It is possible to measure the GPC3 protein in the sample by comparing the result from the sample with the result obtained from the negative control sample not containing GPC3 protein or the result obtained from the positive control sample containing GPC3 protein. Additionally, it is possible to prepare a series of control samples having stepwise changed GPC3 concentrations, and then obtain measurement results for each control sample as numerical values to make a standard curve, and thereby measuring the GPC3 protein in the sample quantitatively based on the standard curve and the measured value of the sample.

A preferable embodiment of measurement of GPC3 protein bound to a support through anti-GPC3 antibody includes a method of using anti-GPC3 antibody labeled with a labeled substance.

For example, a sample is contacted with anti-GPC3 antibody immobilized to a support, and after washing, the sample is measured with use of a labeled antibody specifically recognizing GPC3 protein.

Labeling of the anti-GPC3 antibody can be performed according to any method generally known. As the labeled substance, labeled substances known by a skilled person in the art can be used, such as fluorescent dyes, enzymes, coenzymes, chemiluminescent, and radioactive substances. Specific examples thereof can include radioisotopes (32P, 14C, 125I, 3H, 131I, and the like), fluorescein, rhodamine, dansyl chloride, umbelliferone, luciferase, peroxidase, alkaline phosphatase, β-galactosidase, β-gulcosidase, horseradish peroxidase, glucoamylase, lysozyme, saccharide oxidase, microperoxidase, biotin, and the like. In the case of using biotin as the labeled substance, avidin to which an enzyme such as alkaline phosphatase is bound is preferably added after addition of biotin-labeled antibody. For the binding of the labeled substance and the anti-GPC3 antibody, any known methods such as the glutaraldehyde method, the malleimide method, the pyridyl disulfide method, and the periodic acid method can be used.

Specifically, a solution containing the anti-GPC3 antibody is added to a support such as a plate, the anti-GPC3 antibody is immobilized to the support. After washing the plate, in order to prevent a non-specific binding of protein, it is blocked by, for example, BSA, gelatin, albumin or the like. The plate is washed again, and a sample is added to the plate. After incubation, the plate is washed, and a labeled anti-GPC3 antibody is added to the plate. After suitable incubation, the plate is washed, and the labeled anti-GPC3 antibody remained on the plate is measured. The measurement can be performed according to a method known to a skilled person in the art, for example, in the case of labeling with use of a radioactive substance, the measurement can be performed according to the liquid scintillation or the RIA method. In the case of labeling with use of an enzyme, a substrate is added to the plate, and the enzymatic change of the substrate, for example, coloring can be measured with use of a spectrophotometer. The specific examples of the substrate can include 2,2-azinobis(3-ethylbenzothiazoline-6-sulfonic acid) diammonium salt (ABTS); 1,2-phenylene-diamine (ortho-phenylene-diamine); 3,3',5,5'-tetramethylbenzidine (TME); and the like.
In the case of a fluorescent substance, the measurement can be performed with use of a fluorometer.

A particularly preferable embodiment of GPC3 protein measuring method of the present invention can include a method of using a biotin-labeled anti-GPC3 antibody and avidin.

Specifically, a solution containing the anti-GPC3 antibody is added to a support such as a plate, the anti-GPC3 antibody is immobilized to the support. After washing the plate, in order to prevent a non-specific binding of protein, it is blocked by, for example, BSA. The plate is washed again, and a test sample is added to the plate. After incubation, the plate is washed, and biotin-labeled anti-GPC3 antibody is added to the plate. After suitable incubation, the plate is washed, and avidin bound with enzyme such as alkaline phosphatase or peroxidase is added to the plate. After incubation, the plate is washed, a substrate corresponding to an enzyme bound to avidin is added to the plate, and GPC3 protein is measured by using enzymatic change of the substrate or the like as an index.

Another embodiment of the GPC3 protein measuring method of the present invention can include a method of using one or more type of primary antibodies specifically recognizing GPC3 protein and one or more type of secondary antibodies specifically recognizing the primary antibodies.

For example, a sample is contacted with one or more type of anti-GPC3 antibodies immobilized on a support. After incubation, the support is washed, and GPC3 protein bound after washing is measured with use of the primary anti-GPC3 antibodies and one or more type of secondary antibodies specifically recognizing the primary antibodies. In this case, the secondary antibodies are preferably labeled with a labeled substance.

Another embodiment of the GPC3 protein measuring method of the present invention can include a method of utilizing an agglutination reaction. In the method, GPC3 can be measured with use of a support which sensitized with anti-GPC3 antibody. As the support sensitized with an antibody, any support may be used as long as it is insoluble, stable and does not cause non-specific reaction. For example, it is possible to use latex particles, bentonite, collodion, kaolin, fixed sheep erythrocyte, or the like. It is preferable to use latex particles. As the latex particles, for example, polystyrene latex particles, styrene-butadiene copolymer latex particles, polyvinyl toluene latex particles can be used, and polystyrene latex particles are preferably used. The sensitized particles are mixed with a sample and the mixture is stirred for a certain time period. As anti-GPC3 antibody is contained in the sample at a higher concentration, the agglutination degree of the particles become larger, and therefore GPC3 can be measured by observing the agglutination with the naked eye. In addition, GPC3 can be measured by measuring the turbidity due to agglutination with use of a spectrophotometer or the like.

Another embodiment of the GPC3 protein measuring method of the present invention can include, for example, a method of using a biosensor which utilizes the surface plasmon resonance phenomenon. The biosensor which utilizes the surface plasmon resonance phenomenon can observe interaction between proteins at real time as a surface plasmon resonance signal using a minute amount of protein without labeling. For example, by using a biosensor such as BIAcore (manufactured by Pharmacia), the binding of GPC3 protein and anti-GPC3 antibody can be measured. Specifically, a sample is contacted with a sensor chip to which anti-GPC3 antibody is immobilized, and GPC3 protein bound to anti-GPC3 antibody can be measured as change in surface plasmon resonance signal.

The measurement method of the present invention can be automatically performed with use of various automatic test systems, and can be examined a large number of samples at once.

An object of the present invention is to provide an agent for monitoring seriousness of hepatitis, for example, an agent for presuming and determining the progression from hepatitis or cirrhosis toward hepatoma, wherein the monitoring agent contains at least an anti-GPC3 antibody. In the case where the monitoring agent is based on ELISA method, it may include a support which transforms the antibody into a solid phase, and also include a support to which an antibody is previously bound. In the case where the monitoring agent is based on the agglutination method of using a support such as latex, it may include an antibody-conjugated support thereon. In addition, the monitoring agent may be a kit suitably including a blocking solution, a reaction solution, a reaction terminating solution, a reagent for treating a sample, and the like.

The prediction of the progression from hepatitis or cirrhosis toward hepatoma may be performed by measuring the GPC3 concentration in the serum of a patient. As the GPC3 concentration in the serum is higher, the possibility of progression to hepatoma increases. For example, in the case where the GPC3 concentration in the serum of a patient with chronic hepatitis or cirrhosis is 0.4 ng/mL or more, the high probability of progression to hepatoma is predicted, and in the case of the concentration less than 0.4 ng/mL, the low probability of progression to hepatoma is predicted.

### Examples

The present invention will be specifically described in detail referring to the following examples. However, the present invention is not limited to these examples.

The following materials were used in the Examples described in the present specification.
As a cell producing natural GPC3, HuH-6 which is a cell strain derived from hepatoblastoma was purchased from Riken Cell Bank, and was cultured in 10% FBS/Dulbecco's Modified Eagle Medium (DMEM) (SIGMA CAT# D6429)/penicillin-streptomycin (GIBCO BRL CAT# 15140-122).
Hybridoma was cultured in 10% FBS/RPMI1640/(SIGMA CAT# R-8578)/gentacin (Scheling-plough).

### Example 1: Preparation of natural GPC3

A dish having a diameter of 150 mm was used to large-scale culture of HuH-6 cells, and the supernatant of the culture was collected and purified. The supernatant of the culture was charged in DEAE Sepharose Fast Flow (Amersham CAT# 17-0709-01), washed and then eluted with a buffer containing 500 mMNaCl. Next, after concentration with use of Centriprep-10 (Millipore CAT#4304), it was purified by gel filtration with Superdex 200 HR 10/30 (Amersham CAT# 17-1088-01) to obtain crude GPC3.

GPC3 with high purity was obtained by preparing an affinity column containing monoclonal antibodies produced using the crude GPC3 as an immunogen, charging HuH-6 crude GPC3 in the column, washing with 0.1 M glycine hydrochloric acid at pH 3. 5, then eluting with 4 M MgCl₂ · 6H₂O, and performing dialysis against a solution of 50 mM Tris-HCl (pH 7.5), 150 mM NaCl.

### Example 2: Preparation of recombinant GPC3

A recombinant GPC3 cDNA-expressing plasmid DNA was constructed by use of plasmid DNA containing a full-length human GPC3 cDNA to obtain flag-tagged soluble GPC3. PCR was performed with use of a downstream primer (5'- ATA GAA TTC CAC CAT GGC CGG GAC CGT GCG C -3' (SEQ ID: No 1)) which was designed so as to remove the hydrophobic region in C-terminal (564-580 amino acids) and an upstream primer (5'-ATA GGA TCC CTT CAG CGG GGA ATG AAC GTT C -3' (SEQ ID: No 2)) which was added with EcoRI recognition sequence and Kozak sequence. The obtained PCR fragment (1711 bp) was cloned to pCXND2-Flag. The prepared expression plasmid DNA was introduced to CHO cell DXB11 strain, and CHO strain highly expressing soluble GPC3 was obtained by selection with 500 µg/mL Geneticin.

A large-scale culture of CHO strain highly expressing flag-tagged soluble GPC3 was performed with use of 1700 cm² roller bottle, and the supernatant of the culture was collected and purified. The supernatant of the culture was charged in DEAE Sepharose Fast Flow (Amersham CAT# 17-0709-01), and after washing, it was eluted with a buffer containing 500 mM NaCl. Next purification was performed with use of Anti-Flag M2 agarose affinity gel (SIGMA CAT#A-2220). Elution was performed with 200 µg/mL of FLAG peptide. After concentration with Centriprep-10 (Millipore CAT#4304), gel filtration with Superdex 200 HR 10/30 (Amersham CAT# 17-1088-01) was performed to remove FLAG peptide. Finally, the eluate was concentrated with use of DEAE Sepharose Fast Flow column and eluted with phosphate buffer (containing 500 mM NaCl) without Tween 20 simultaneously to perform buffer substitution.

### Example 3: Preparation of anti-GPC3 monoclonal antibody

Five Balb/C mice (CRL) were immunized with recombinant GPC3 or natural GPC3. In primary immunization, immunogen was prepared at 100 µg/mouse in case of the recombinant GPC3 and at 10 µg/mouse in case of the natural GPC, emulsified with FCA (Freund's complete adjuvant: H37 Ra Difco (3113-60) or Becton Dickinson (cat#231131)), and administrated subcutaneously. After two weeks, booster was performed by preparing immunogen at 50 µg/mouse in case of the recombinant GPC3 and at 5 µg/mouse in case of the natural GPC3, then emulsifying them with FIA (Freund's incomplete adjuvant: Difco (0639-60) or Becton Dickinson (cat#263910)), and administrating them subcutaneously. Thereafter, booster was performed five times at intervals of one week. Final immunization was performed by administering the protein into tail vein in the same amount as in the booster. After confirming the saturation of antibody titer in a serum against GPC3 by ELISA using immunoplate coated with GPC3 core protein, mouse myeloma cell P3U1 and mouse splenocyte were mixed to perform cell fusion using PEG1500 (Roche Diagnostics, cat#783641). The cells were inoculated to 96-well culture plate, and from next day, Selective culture was performed in HAT medium and thereafter the supernatant of the culture was screened with ELISA. For positive clones, they were subjected to monocloning according to a limiting dilution method, and the culture supernatant in an extensive culture was collected. In screening with ELISA, binding activity to GPC3 core protein was used as an index, and an anti-GPC3 monoclonal antibody having a strong binding ability was obtained.

The purification of the antibody was performed with use of Hi Trap Protein G HP (Amersham CAT#17-0404-01). The supernatant of hybridoma culture was directly charged in a column, washed with a binding buffer (20mM sodium phosphate (pH 7.0)), and then eluted with an elution buffer (0.1M glycine-HCl (pH 2.7)). The elution was collected in a tube containing a neutralization buffer (1M Tris-HCl (pH 9.0)) to neutralize immediately. After antibody fractions were pooled, dialysis was performed with 0.05% Tween 20/PBS over one day to substitute buffer. The solution of purified antibody was added with NaN₃ until its concentration reached 0.02%, and thereafter it was kept at 4°C.

### Example 4: Isotyping of anti-GPC3 monoclonal antibody

The antibody concentration was quantitatively measured by performing mouse IgG sandwich ELISA using goat anti-mouse IgG (gamma) (ZYMED CAT# 62-6600) and alkaline phosphatase-goat anti-mouse IgG (gamma)(ZYMED CAT# 62-6622), and using commercially available purified mouse IgGl antibody (ZYMED CAT#02-6100) as a standard.
The isotyping of anti-soluble GPC3 monoclonal antibody was performed with use of ImmunoPure Monoclonal Antibody Isotyping Kit II (PIERCE CAT# 37502) according to a method indicated in a manual attached thereto. As the result of the isotyping, all antibodies were IgG1 type.

### Example 5: Identification of recognition site of anti-GPC3 monoclonal antibody

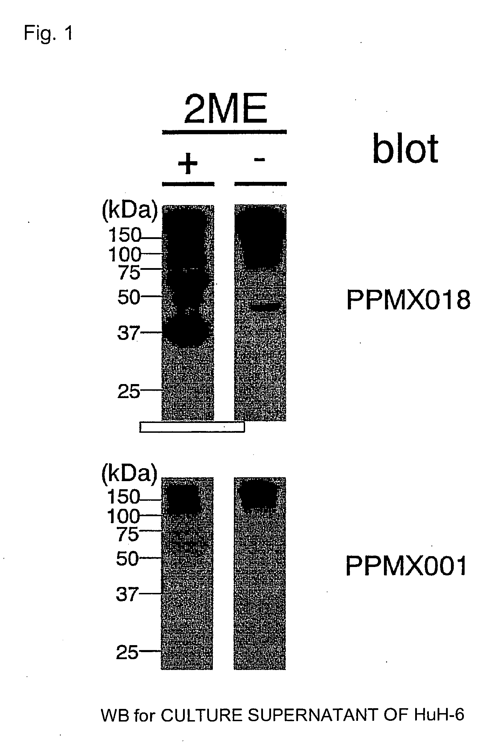

The outline of western blot was follows: HuH-6 hepatoma cell strain was cultured in DMEM +10% FBS for 5 days, its culture supernatant was applied at 5 µl/lane, and SDS-polyacrylamide gel electrophoresis was performed under reduction and non-reduction conditions. Next, protein was transferred to Hybond P membrane (manufactured by Amersham Bioscience Corporation), and after blocking a transfer membrane, to which each antibody was reacted. Next, HRP labeled anti-mouse antibody was reacted, and after adding ECL detection reagent (manufactured by Amersham Bioscience Corporation) the obtained chemiluminescence was detected by exposing X-ray film thereto. From the result of western blot shown in Fig. 1, it can be determined that PPMX018 is an N-terminal recognizing antibody and PPMX001 is a C-terminal recognizing antibody. The result of studying in the same manner indicated PPMX013 and M18D04 (FERM BP-10325) as the N-terminal recognizing antibody.
Hybridoma for producing M18D04 was deposited in International Patent Organism Depositary, National Institute of Advanced Industrial Science and Technology; Tsukuba Central 6, 1-1-1, Azuma, Tsukuba, Ibaragi, Japan (ZIP CODE 305-8566).

### Example 6: Preparation of anti-GPC3 polyclonal antibody

Recombinant GPC3 or natural GPC3 was used to prepare guinea pig polyclonal antibody. A known method was use for this preparation. GPC3 emulsified with an adjuvant was administered subcutaneously to perform immunization. The administration was repeated several times, and after confirming that antibody titer increased, blood was collected to obtain a serum, and thereafter polyclonal antibody was obtained by ammonium sulfate precipitation.

### Example 7: Establishment of sandwich ELISA system

In order to measure soluble GPC3 in blood, a number of sandwich ELISA system using GPC3 was established and from which a measurement system having excellent sensitivity and specificity was selected. In order to achieve high measurement sensitivity in coloring, TMB from Scytek Laboratories, Inc. was used.

Ninety six-well immunoplate was coated with anti-GPC3 monoclonal antibody or polyclonal antibody diluted with PBS(-) to 10 µg/mL, and it was incubated at 4°C over night. In the next day, after it was washed with 300 µL/well of PBS three times, 150 µL of the immunoassay stabilizer from ABI Inc. (ABI #10-601-001) was added to it to perform blocking. After maintaining several hours at room temperature, or at 4°C over night, purified protein, human serum and the like suitably diluted with a dilution buffer (50mM Tris-Cl pH 8.0, 0.15M NaCl) containing an animal serum and the like were added to it , and incubated for 2 hours at room temperature. Next, a C-terminal region or N-terminal region recognizing antibody which was labeled with biotin and diluted with PBS (-) containing an animal serum and the like to 20 µg/mL was added thereto and the mixture was incubated for 2 hours at room temperature. After discarding the reaction solution, streptavidin-HRPO from Vector Laboratories (Vector #SA-5004) which was diluted to 3 µg/mL with Stab-ELISA-r from Cygnus Technologies, Inc (Cygnus #I-030) containing a suitable amount of an animal serum was added, and the mixture was incubated for 0.5 hours at room temperature. After washing with 300 µL/well of a washing buffer 5 times, according to the protocol attached by Scytek Laboratories, Inc., the mixture was colored with TMB (Cat#TM4999), and its absorbance was measured with a microplate reader.

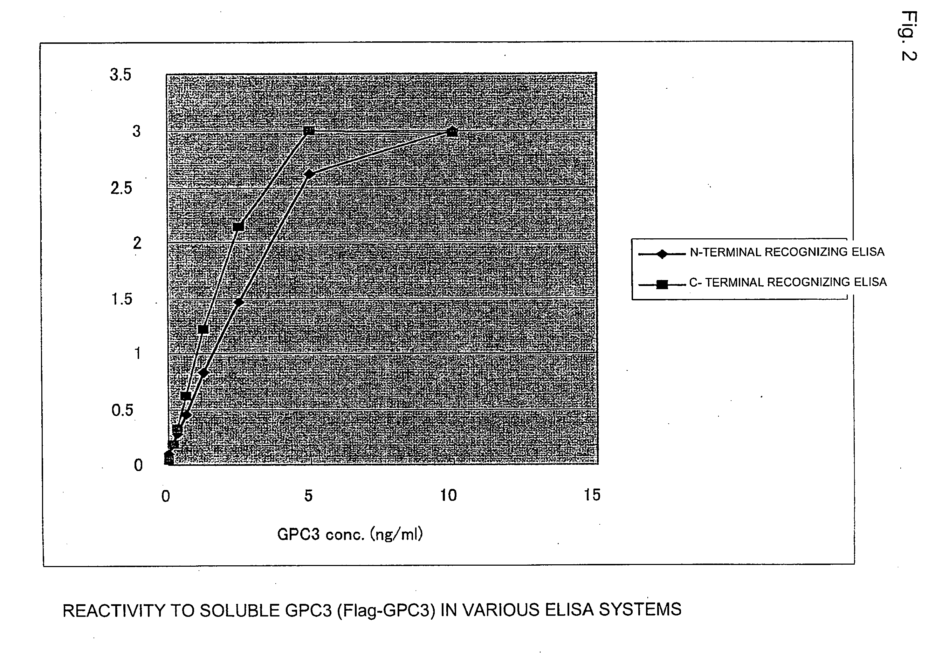

For the biotination of the antibody, Sulfo-NHS-LC-Biotin (CAT# 21335) from Pierce Biotechnology was used. In addition, for the conversion to GPC3 concentration in a sample, a spreadsheet software, GlaphPad PRISM (GlaphPad software Inc. ver.3.0) was used to analyze. Fig. 2 shows standard curves obtained by using recombinant GPC3 in ELISA measurement systems which were constructed using PPMX001 and PPMX013, antibodies recognizing C-terminal region, and using PPMX018 and PPMX013, antibodies recognizing N-terminal region. In the C-terminal region recognizing measurement system, higher absorbance could be obtained comparing with that in the N-terminal region recognizing measurement system. In the former measurement system, it is presumed to recognize full-length GPC3 and C-terminal peptide, while in the latter measurement system, it is presumed to recognize full-length GPC3 and N-terminal peptide.

### Example 8: Measurement of GPC3 in HuH-6 culture solutions and in serum of hepatoma patients in measurement system using antibodies recognizing C-terminal region and those recognizing N-terminal region

Two ELISA measurement systems used for making the above-described standard curves were used to measure concentrations of GPC3 purified from the HuH-6 culture supernatant and GPC3 in the serum of 20 hepatoma patients. The results of this experiment were unexpected and different from the above results of the standard curves (see, Table 1) . That is, when using measurement systems in which the GPC3 recognition sites were different, the obtained measured values were largely different among recombinant GPC3, HuH-6-derived natural GPC3, and GPC3 in the serum of patients. In the measurement system for recognizing C-terminal region, it was presumed that the large difference between the recombinant GPC3 and the GPC3 in the patient serum was caused due to a difference in the contents of heparin sulfate added to the C-terminal region of GPC3.

**[Table 1]**

| Results of measuring GPC3 derived from HuH-6 and in serum of hepatoma patient by ELISA system using N-terminal recognizing antibodies and C-terminal recognizing antibodies | | | |
|---|---|---|---|
| | | N-terminal recognizing ELISA | C-terminal recognizing ELISA |
| HuH-6 | 10 | 3.000 | 1.211 |
| GPC3 | 5 | 2.720 | 0.638 |
| conc. | 2.5 | 1.599 | 0.334 |
| (ng/ml) | 1.25 | 0.792 | 0.187 |
| | 0.63 | 0.467 | 0.114 |
| | 0.31 | 0.258 | 0.077 |
| | 0.16 | 0.175 | 0.060 |
| | 0 | 0.103 | 0.041 |
| Patients | 1 | 0.737 | 0.343 |
| No. | 2 | 0.679 | 0.030 |
| | 3 | 0.577 | 0.083 |
| | 4 | 0.488 | 0.105 |
| | 5 | 0.354 | 0.078 |
| | 6 | 0.236 | 0.026 |
| | 7 | 0.192 | 0.031 |
| | 8 | 0.153 | 0.038 |
| | 9 | 0.139 | 0.042 |
| | 10 | 0.137 | 0.027 |
| | 11 | 0.139 | 0.024 |
| | 12 | 0.127 | 0.062 |
| | 13 | 0.130 | 0.049 |
| | 14 | 0.116 | 0.035 |
| | 15 | 0.110 | 0.021 |
| | 16 | 0.100 | 0.033 |
| | 17 | 0.105 | 0.036 |
| | 18 | 0.077 | 0.020 |
| | 19 | 0.073 | 0.017 |
| | 20 | 0.058 | 0.022 |

### Example 9: Measurement of GPC3 concentration in serum of healthy individuals, and patients with chronic hepatitis, cirrhosis or hepatoma with use of measurement system recognizing N-terminal region.

Among various ELISA measurement systems for recognizing N-terminal region, the measurement system using M18D04 (FERM BP-10325) monoclonal antibody and guinea pig polyclonal antibody having excellent sensitivity was used to measure the serum GPC3 concentration in healthy individuals, and patients with chronic hepatitis, cirrhosis or hepatoma. As the result, positive rates of the serum GPC3 with cut off value of 0.4 were 1.5% in healthy individuals, 36% in chronic hepatitis, 51.4% in cirrhosis, and 51% in hepatoma patients. Furthermore, for three hepatoma patients who have been admitted to a hospital, their serum were periodically sampled for 8 years before onset of hepatoma to measure serum GPC3 concentration, and it was found that as a liver disease progressed, blood GPC3 concentration tended to increase. These results demonstrated that the present measurement system is useful for monitoring seriousness of hepatitis rather than for using as an agent for diagnosing a hepatoma.

**[Table 2]**

| Results of measuring serum GPC3 by ELISA system using N-terminal recognizing antibodies in patients with hepatoma, cirrhosis or chronic hepatitis, and in healthy individuals | | | | | | | | | |
|---|---|---|---|---|---|---|---|---|---|
| | | Normal(n=200) | | CH(n=172) | | LC(n=35) | | HCC(n=96) | |
| | | No. of Positive | Positive rate (%) | No. of Positive | Positive rate (%) | No. of Positive | Positive rate (%) | No. of Positive | Positive rate (%) |
| GPC3 conc. (ng/ml) | >0.4 | 3 | 1.5 | 62 | 36 | 18 | 51.4 | 49 | 51 |
| | 0.2-0.4 | 88 | 44 | 31 | 18 | 8 | 22.8 | 21 | 21.8 |
| | <0.2 | 109 | 54.5 | 79 | 46 | 9 | 25.8 | 26 | 27.2 |

### Example 10: Immunostaining of tissues derived from healthy individuals, chronic hepatitis, and cirrhosis

Immunostaining with anti-GPC3 monoclonal antibody was performed on the histopathogical specimens obtained from biopsy in chronic hepatitis and cirrhosis patients.
Each sample was prepared by slicing a specimen fixed and embedded in paraffin 4 µm-thick, then mounting them on a slide glass, and was left at 37°C for 16 hours to dry sufficiently. The paraffin was removed from the sample by immersing in 100% xylen for 5 minutes 3 times, and the sample was hydrophilized by immersing in 100% alcohol for 5 minutes 3 times and in70% ethanol for 5 minutes. Thereafter, the sample was washed with 50mM TBS for 5 minutes 3 times, and then an antigen was activated in a citric acid buffer (10mM, pH 6.0) at 120°C for 10 minutes. After the activation of the antigen, the sample was washed with TBS for 5 minutes 3 times, and then reacted with anti-GPC3 antibody diluted to 1 µg/mL at room temperature for one hour. In order to inactivate endogenous peroxidase, the sample was exposed to 0.3% hydrogen peroxide for 15 minutes at room temperature. Further, after washing with TBS 3 times, the sample was reacted with ENVISION+Kit/HRP (DAKO), a secondary antibody, for one hour. After washing with TBS for 5 minutes 3 times, DAB (3,3'-Diaminobenzidine Tetrahydrochloride) was used for a coloring substrate. Hematoxylin was also used for counter staining of nucleus.

As the result of immunohistochemistry in chronic hepatitis and cirrhosis, it was found that GPC3 was positive in both diseases, and a staining image thereof was observed on cell membranes. On the other hand, the staining of GPC3 was not observed in a normal liver tissue.
Accordingly, it was determined that the measurement of the GPC3 concentration was useful for monitoring seriousness of hepatitis.

## Claims

1. An agent for monitoring seriousness of hepatitis, wherein the agent comprises an anti-GPC3 antibody.

2. The agent for monitoring according to claim 1, wherein the anti-GPC3 antibody is an antibody recognizing an N-terminal region of GPC3.

3. The agent for monitoring according to claim 1 or 2, which presumes progression of hepatitis or cirrhosis toward hepatoma.

4. The agent for monitoring according to any one of claims 1 to 3, wherein the anti-GPC3 antibody immobilized on a support and the anti-GPC3 antibody labeled by a labeled substance are used.

5. A method of monitoring seriousness of hepatitis, comprising measuring GPC3 in a sample using an anti-GPC3 antibody.

6. The method of monitoring according to claim 5, wherein the anti-GPC3 antibody is an antibody recognizing an N-terminal region of GPC3.

7. The method of monitoring according to claim 5 or 6, wherein the sample is blood, serum or plasma.

8. The method of monitoring according to any one of claims 5 to 7, which presumes progression of hepatitis or cirrhosis toward hepatoma.

9. The method of monitoring according to any one of claims 5 to 8, wherein the anti-GPC3 antibody immobilized on a support and the anti-GPC3 antibody labeled by a labeled substance are used.
